(19) Europäisches Patentamt
European Patent Office
Office européen des brevets

(11) Veröffentlichungsnummer: **0 025 218**
**B1**

(12) # EUROPÄISCHE PATENTSCHRIFT

(45) Veröffentlichungstag der Patentschrift: **18.05.83**

(51) Int. Cl.³: **C 07 C 103/52,** A 61 K 37/02

(21) Anmeldenummer: **80105256.4**

(22) Anmeldetag: **04.09.80**

(54) **Pharmakologisch aktive Peptide und diese enthaltende Arzneimittel.**

(30) Priorität: **06.09.79 DE 2936099**

(43) Veröffentlichungstag der Anmeldung: **18.03.81 Patentblatt 81/11**

(45) Bekanntmachung des Hinweises auf die Patenterteilung: **18.05.83 Patentblatt 83/20**

(84) Benannte Vertragsstaaten: **AT BE CH DE FR GB IT LI LU NL SE**

(56) Entgegenhaltungen:

**UNLISTED DRUGS-Feb. 80, Edit. (Special Library Association) Naunym Schmied Arch Pharmacol 308 (Suppl): R 39 (155 Abs), 1979 casomorphins**
**UNLISTED DRUGS-Oct. 80, Edit. (Special Library Association) Naunyn-Schmiedeberg's Arch Pharmacol 311 (Suppl): 266 Abs. März 80 beta-CasM-4**

(73) Patentinhaber: **Brantl, Victor, Dipl.-Chem., Frauenplatz 10, D-8000 München 2 (DE)**
Patentinhaber: **Teschemacher, Hansjörg, Prof. Dr., Frankfurter Strasse 107, D-6300 Giessen 1 (DE)**
Patentinhaber: **Henschen, Agnes, Dr., Am Klopferspitz, D-8033 Martinsried (DE)**
Patentinhaber: **Lottspeich, Friedrich, Dr., Am Klopferspitz, D-8033 Martinsried (DE)**

(72) Erfinder: **Brantl, Victor, Dipl.-Chem., Frauenplatz 10, D-8000 München 2 (DE)**
Erfinder: **Teschemacher, Hansjörg, Prof. Dr., Frankfurter Strasse 107, D-6300 Giessen 1 (DE)**
Erfinder: **Henschen, Agnes, Dr., Am Klopferspitz, D-8033 Martinsried (DE)**
Erfinder: **Lottspeich, Friedrich, Dr., Am Klopferspitz, D-8033 Martinsried (DE)**

(74) Vertreter: **Schacht, Wilhelm, Dr. jur., Am Eselsweg 47, D-6500 Mainz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Pharmakologisch aktive Peptide und diese enthaltende Arzneimittel

Die Erfindung betrifft neue pharmakologisch aktive Peptide, insbesondere opiatartig wirkende, Verfahren zu deren synthetischer Herstellung, sowie nach diesen Verfahren hergestellte pharmakologisch aktive Peptide.

Es sind pharmakologisch aktive Peptide bekannt, welche in besonderen biologischen Testsystemen eine spezifische opiatartige Wirkung entfalten. Es sind dies insbesondere die von Hughes et al. (Nature 258, 577, 1975) aus dem Gehirn isolierten Pentapeptide: das Methionin- und das Leucinenkephalin sowie deren längerkettige Analoga. Diese bekannten Peptide und deren synthetische Derivate weisen den Nachteil auf, dass diese nach in vitro Behandlung mit Proteasen (z.B. Pronase E, Merck) nach kurzer Incubationszeit ihre pharmakologische Wirkung, in diesem Fall eine opiatartige, verlieren. Bei in vivo Verabreichung werden diesen Peptide durch körpereigene Proteasen leicht angegriffen und zerstört.

Auf der Tagung der int. Narcotic Research Conference im Juni 1979, North Falmouth, USA, sind neue Peptide, β-Casomorphine beschrieben worden, welche eine neuartige Sequenz aufweisen, welche überraschenderweise aufgrund dieser Sequenz eine erhöhte Stabilität gegenüber Proteasen (z.B. Pronase E, Merck) aufweisen. Diese Peptide zeigen eine starke opiatartige Wirkung an verschiedenen bekannten Testsystemen (Rezeptorbindungstest und isolierter Meerschweinchendarm). Nach Injektion von 0.35 μmolen β-Casomorphin-5 (Tyr-Pro-Phe-Pro-Gly) in das Ventrikelsystem des Gehirns von Ratten zeigt sich eine starke, opiatspezifische Analgesie, welche innerhalb von 10 Minuten ihre maximale Wirkung erreicht hat und nach 60 Minuten völlig abgeklungen ist. Diese im Vergleich zu Morphin kurze Wirkungsdauer, bei gleicher Analgesie, deutet bereits auf einen möglichen völligen oder teilweisen Abbau der Substanz durch Enzyme des Gehirns hin.

Nach parenteraler oder oraler Applikation der Substanz zeigt das β-Casomorphin-5, welches übrigens das bisher stärkste bekannte opiatartig wirkende Peptid der β-Casomorphinfamilie ist, keine analgetische Wirkung. Die Dosis betrug bei diesen Versuchen bis zu 150 mg pro Kg Körpergewicht der Ratten. Dieser Nachteil der bekannten Peptide kann ganz oder teilweise auf die Inaktivierung der Substanz im Blut/oder im Gehirn zurückgeführt werden. Inkubationsversuche der Substanz mit Rattengehirnhomogenat oder Blutplasma zeigten, dass schon nach kurzer Inkubationszeit von 15 Minuten die opiatartige Wirkung verschwindet.

Aufgabe der Erfindung ist es, neue pharmakologisch aktive Peptide zu schaffen, β-Casomorphine, Analoga und/oder Derivate, welche nach Inkubation (bis 120 Minuten) mit Rattengehirnhomogenat und/oder -homogenatüberstand und/oder Blutplasma (siehe Ausführungsbeispiel) ihre pharmakologische Wirkung, zum Beispiel die opiatartige, beibehalten. Aufgabe der vorliegenden Erfindung ist es ferner, pharmakologisch aktive Peptide, β-Casomorphine, Analoga und/oder Derivate zu schaffen, welche nach parenteraler (z.B. intravenöser oder subcutaner Injektion) und/oder oraler Applikation eine pharmakologische Wirkung, insbesondere eine opiatartige zeigen. Aufgabe der Erfindung ist weiterhin, den Fachmann durch die Offenbarung der Aminosäuresequenz, insbesondere der Position der D-Aminosäure, in die Lage zu versetzen, mit üblichen Methoden das Peptid in beliebiger Menge herzustellen.

Diese Aufgabe ist gemäss der Erfindung dadurch gelöst, dass die pharmakologisch aktiven Peptide folgende Formeln aufweisen:

$Tyr^1$-X-A-Pro-B-Pro-C.....

wobei Tyr gleich der Aminosäure Tyrosin und Pro gleich der Aminosäure Prolin ist. A, B, C..... ist jede beliebige Aminosäure der stereochemischen L-Form. X ist jede beliebige Aminosäure der D-Form.

Nach einer Weiterbildung der Erfindung weisen die pharmakologisch aktiven Peptide folgende Formeln auf:

$Tyr^1$-X-A
$Tyr^1$-X-A-Pro
$Tyr^1$-X-A-Pro-B
$Tyr^1$-X-A-Pro-B-Pro
$Tyr^1$-X-A-Pro-B-Pro-C
$Tyr^1$-X-A-Pro-B-Pro-C-Pro
$Tyr^1$-X-A-Pro-B-Pro-C-Pro-D.....

Hierbei kann A, B, C, D..... jede beliebige L-Aminosäure sein, auch Prolin und Tyrosin. X kann jede beliebige D-Aminosäure sein.

Nach einer anderen Weiterbildung der Erfindung bedeuten die Buchstaben A, B, C, D der aufgeführten Peptide:

X: D-Prolin
A: L-Phenylalanin
B: L-Glycin
C: L-Isoleucin
D: L-Asparagin.

Nach einer besonders vorteilhaften Weiterbildung der Erfindung ist X durch die D-Aminosäuren: D-Alanin, D-Threonin oder D-Valin ersetzt.

Nach einer anderen Weiterbildung ist

a) das endständige L-Tyrosin der allgemeinen Formel:

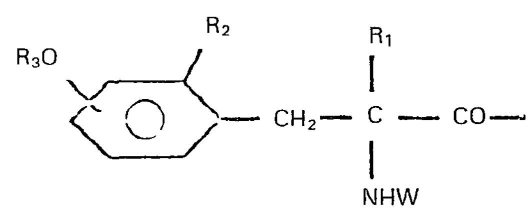

durch

$R_1$ für Wasserstoff oder eine Alkylgruppe mit 1 bis 4 C-Atomen,

$R_2$ für Wasserstoff oder zusammen mit $R_1$ für eine Äthylenbrücke,

$R_3$ für Wasserstoff, eine Alkylgruppe mit 1 bis 4 C-Atomen oder eine $R_4CO$-Gruppe,

$R_4$ für einen gesättigten oder ungesättigten, geradkettigen oder verzweigten Alkylrest mit 1 bis 17 C-Atomen, einen Phenylrest

oder einen Phenylalkylrest mit 7 bis 12 C-Atomen, wobei die Phenylreste durch 1 oder 2 Substituenten aus der Reihe Halogen, Alkyl mit 1 bis 4 C-atomen oder Alkoxy mit 1 bis 4 C-Atomen substituiert sein können,

wobei die $R_3O$-Gruppe sich in meta- oder para-Stellung zum

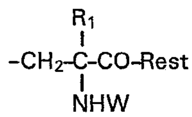 befindet,

W für Wasserstoff, Alkyl mit 1 bis 5 C-Atomen, Alkenyl mit 3 bis 5 C-Atomen, Cyclopropylmethyl, Cyclobutylmethyl, $R_4CO$-, H-Lys-, H-phe- oder H-Thyr, ersetzt.

b) das L-Phenylalanin der allgemeinen Formel:

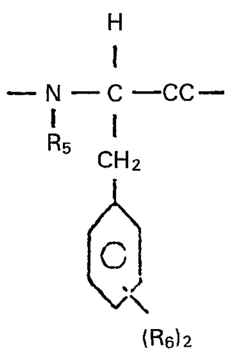

durch

$R_5$ für Wasserstoff oder Alkyl mit 1 bis 4 C-Atomen,

$R_6$ für Wasserstoff, Fluor, Chlor, Brom, Nitro, Alkyl mit 1 bis 4 C-Atomen oder Alkoxy mit 1 bis 4 C-Atomen,

Z für 1 oder 2

ersetzt.

c) das L-Prolin (an den Positionen der Aminosäure 4, 6, 8..... des Peptids) der allgemeinen Formel:

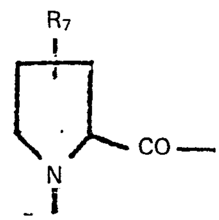

so modifiziert, dass:

$R_7$ für Wasserstoff, eine Hydroxygruppe, eine Alkyl- oder Alkoxygruppe mit 1 bis 4 C-Atomen steht,

— am Stickstoff eine Alkyl- oder Alkoxygruppe mit 1 bis 4 C-Atomen gebunden ist,

— eine oder mehrere Ketogruppen in den Ring eingeführt sind.

d) die endständige, C-terminale Aminosäure ein Amid oder Ester.

Die erhöhte Stabilität der erfindungsgemässen Peptide gegenüber abbauenden Enzymen beruht auf den Einbau einer D-Aminosäure in Position 2 des Peptids. Dieser Einbau war völlig fernliegend, da die Entdecker und Erstbeschreiber dieser Verbindungsklasse der β-Casomorphine auf der Internationalen Narcotic Research Conference, Juni 1979, North Falmouth, USA, berichteten, dass durch die alternierende Prolinsequenz eine völlig überraschende Stabilität gegenüber Peptidasen, z.B. Pronase E, Merck resultiert. So lag es also völlig fern eine weitere Stabilisierung gegen Proteasespaltung vorzunehmen. Die D-Aminosäure in Position 2 des Peptids verhindert den Abbau durch natürliche Enzyme, da die natürlichen Enzyme bevorzugt Peptidbindungen zwischen L-Aminosäuren spalten.

Nach einer anderen Weiterbildung der Erfindung ist die C-terminale Aminosäure eines ungeradzahligen Peptids Methionin.

Besonders vorteilhaft ist die Aminosäure Methionin in C-terminaler Position bei Penta, Hepta und Nonapeptiden.

(die C-terminale Aminosäure steht immer rechts vom Tyr[1])

Sehr günstig ist die Derivatisierung des Methionins. Dabei liegt das C-terminale Methionin als -Met(O)-OH vor:

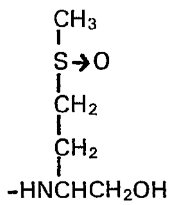

Diese Derivatisierung erbringt eine Verstärkung der opiatartigen Wirkung mit sich, das heisst mit kleineren Dosen der Peptide können stärkere Wirkungen erzielt werden.

Nach einer Weiterbildung der Erfindung werden die erfindungsgemässen pharmakologisch aktiven Peptide mit einem in der Peptidchemie und/oder in der Natur üblichen Verfahren synthetisiert.

Unter üblichen Verfahren wird das Einführen sowie das Abspalten von Schutzgruppen (z.B. der Schutz der Aminogruppe durch Carbobenzoxychlorid; der Schutz von Säuregruppen als Ester) während der Synthese verstanden. Üblich sind ebenfalls Synthesen an Trägermaterialien wie z.B. Polystyrol.

Nach einer Weiterbildung der Erfindung werden die pharmakologisch aktiven Peptide durch Knüpfen der Peptidbindung zwischen den einzelnen Aminosäuren und/oder den kleineren Peptidfragmenten mit den üblichen Methoden erzeugt.

Arzneimittel und Tierarzneimittel, dadurch gekennzeichnet, dass diese, die in den Ansprüchen 1-10 beschriebenen pharmakologisch aktiven Peptide und/oder deren Derivate und/oder deren Säureadditionssalze und/oder deren Metallkomplexe enthalten.

Diese Säureadditionssalze können vorzugsweise Hydrochloride, Lactate oder Citrate sein. Als Metallkomplexe können Zink-, Magnesium- und Cobaltverbindungen verwendet werden. Als Derivate sind sämtliche Verbindungen zu verstehen, die als Grundgerüst die erfindungsgemässe Peptidsequenz der

L-Aminosäuren mit Einbau einer D-Aminosäure in der Position 2 aufweisen und durch Substitution einzelner anhängender Gruppen Variationen erzielt werden. Die dabei erzielte positive oder negative Auswirkung auf die jeweils gewünschte Wirkung kann dann in den jeweiligen Tests einfach festgestellt werden.

Diese Arzneimittel können insbesondere als Antitussiva, Antidiarrohica, Analgetica, Antipsychotica, Tranquilizer Verwendung finden.

*Beispiel*

Synthese eines erfindungsgemässen Pentapeptids der Sequenz:

Tyr-D-Ala-Phe-Pro-Gly-Methylester und

Tyr-D-Pro-Phe-Pro-Gly-Methylester

A Syntheseplan: Die Aminokomponente wird bei −15°C oder niedriger in Dimethylformamid (DMF) mit einem 0,5 molaren Überschuss an gemischtem Anhydrid einer Z-Aminosäure-isobutylcarbonsäure (wobei Z als Schutzgruppe dient und einen n-Benzyloxycarbonyl Rest darstellt) 2 - 4 Stunden umgesetzt.

Das gemischte Anhydrid wird in DMF bei −15°C oder niedriger in 10 bis 15 Minuten unter Einsatz eines 6%igen Überschusses am Z-Aminosäurederivat und N-Methylmorpholin über den Chlorameisensäureisobutylester gebildet. Der Überschuss an gemischtem Anhydrid wird dann zerstört. Bei 0°C wird der pH des Reaktionsproduktes mit wässriger, gesättigter $KHCO_3$ Lösung auf 8 eingestellt und 30 Minuten bei 0°C gerührt.

Die Peptide wurden mit Ethylacetat extrahiert. Das Ethylacetat/Peptidgemisch wurde, um dass Z-Aminosäure-Kaliumsalz zu entfernen, 3mal mit Natriumchlorid/Wasser, 3mal mit Wasser gewaschen und abgedampft. Das so erhaltene Peptid, welches noch die Schutzgruppe Z trägt, wurde in Methanol hydriert. Dabei wurden 100 - 500 mg Pd/Aktivkohle Katalysator pro mmol Peptid zugesetzt. Die $CO_2$-Abspaltung wurde mit Ba $(OH)_2$ Lösung kontrolliert. Der Katalysator wurde abfiltriert (Schleicher & Schüll Papierfilter N° 595), mit Wasser ausgiebig gewaschen, und das Filtrat auf einem Rotationsverdampfer (Büchi, Rotavapor RE) eingedampft. Das gewünschte deblockierte Peptid ist im Rückstand.

B Synthese des Pentapeptids

Tyr-D-Ala-Phe-Pro-Gly-Methylester und

Tyr-D-Pro-Phe-Pro-Gly-Methylester

Stufe 1: a) Herstellung des gemischten Anhydrids. (Z-Phe-Pro-gemischtes Anhydrid)

640 mg (1,6 mM; = 6%iger Überschuss) des Dipeptids Z-L-Phe-L-Pro (wobei Z ein N-Benzyloxycarbonyl Rest ist, welcher als Schutzgruppe fungiert) werden in 20 ml Dimethylformamid (DMF) mit 200 µl (1,5 mM) Chlorameisensäureisobutylester bei −15°C nach Zusatz von 170 µl (1,6 mM) N-Methylmorpholin 15 Minuten umgesetzt.

b) Vorbereitung der Aminokomponente.

125,6 mg (1,0 mM) Glycinmethylesterhydrochlorid werden in 20 ml DMF unter Zusatz von 110 µl (1 mM) N-Methylmorpholin bei −15°C aufgelöst.

Stufe 2: Umsetzung des gemischten Anhydrids der Stufe 1 a) mit der Aminokomponente 1 b). Das Z-Phe-Pro-gemischte Anhydrid wird mit dem Glycinmethylester in 40 ml DMF bei −15°C 4 Stunden umgesetzt.

$$\text{Z-Phe-Pro-gem. Anhydrid + Gly-Metester} \xrightarrow[4\ h]{-5°C} \text{Z-Phe-Pro-Gly-Metester.}$$

Vor der Aufarbeitung wird der 50%ige Überschuss an gemischtem Anhydrid zerstört. Bei 0°C wird der pH des Reaktionsproduktes mit wässriger, gesättigter $KHCO_3$ Lösung auf pH 8 eingestellt und 30 Minuten bei 0°C gerührt.

Danach wird das Peptid mit 50 - 100 ml Ethylacetat (EtAc) extrahiert; das EtAc/Peptidgemisch wird mit gesättigter, wässriger Natriumchlorid Lösung gewaschen. Nach einem weiteren abschliessenden Waschen mit Wasser wird die EtAc-Phase eingedampft.

Stufe 3: Abspalten der Schutzgruppe durch Hydrierung.

Das Peptid wird in 30 ml Methanol gelöst und 100 mg Palladium auf Aktivkohle (Merck) zugegeben. Nach dem Verdrängen der Luft durch Stickstoff wird in das Reaktionsgefäss Wasserstoff eingeleitet. Die Hydrierung wird bei 25°C - 30°C durchgeführt. Die Hydrierung ist beendet, wenn kein $CO_2$ mehr freigesetzt wird, d.h. wenn nach Kontrolle in wässriger Bariumhydroxid Lösung kein Niederschlag mehr gebildet wird. Die Lösung wird filtriert, mit Wasser gewaschen und am Rotationsverdampfer einrotiert. Dieses verbleibende Zwischenprodukt wird dann in Stufe 4 als Aminokomponente eingesetzt.

Stufe 4: a) Herstellung des gemischten Anhydrids Z-D-Ala- und Z-D-Pro-gem. Anhydrid

334,8 mg Z-D-Ala (entsprechend auch Z-D-Pro) werden in 15 ml DMF unter Zusatz von 170 µl (1,5 mM) N-Methylmorpholin gelöst und mit 180 µl (1,4 mM) Chlorameisensäureisobutylester bei −15°C 15 Minuten umgesetzt.

b) Umsetzung des gemischten Anhydrids aus der Stufe 4 a) mit der Aminokomponente der Stufe 3. Z-D-Pro oder Z-D-Ala-Anhydrid + Phe-Pro-Gly-

$$\text{Metester (in 15 ml DMF)} \xrightarrow[4\ h]{-15°C}$$

Z-D-Ala-Phe-Pro-Gly-Methylester und
Z-D-Pro-Phe-Pro-Gly-Methylester
(Die Herstellung des Z-D-Ala und des Z-D-Pro ist am Ende der Synthese aufgeführt.)

Die Zerstörung des überschüssigen gemischten Anhydrids, die Extraktionsschritte, die Hydrierung werden identisch wie vorher beschrieben durchgeführt.

Das Endprodukt 4 b) dient als Aminokomponente für die Stufe 5. Eine Kontrolle der Aminosäurezusammensetzung nah Hydrolyse ergab die richtige molare Aminosäurerelation dieses Peptids. Ein Test auf die Opiataktivität (Testmethode siehe Beispiel I) ergab keinerlei opiatartige Wirkung.

Stufe 5: a) Bildung des gemischten Anhydrids (Z-Tyr-gem. Anhy.) 629, 24 (1,4 mM) N,O-di-Z-L-Tyrosin werden in 15 ml DMF unter Zusatz von 165 µl (1,4 mM) N-Methylmorpholin gelöst und mit 175 µl (1,3 mM) Chlorameisensäureisobutylester bei −15°C 15 Minuten umgesetzt.

5: b) Umsetzung des gemischten Anhydrids aus

Stufe 5 a) mit dem Endprodukt der Stufe 4 b).

Das Endprodukt der Stufe 4 b) wird in 15 ml DMF gelöst und mit dem gemischten Anhydrid der Stufe 5 a) bei −15°C, 4 Stunden umgesetzt. Die Zerstörung des überschüssigen gemischten Anhydrids, die Extraktion und die Hydrierung erfolgen wie oben beschrieben.

Eine nachfolgende Auftrennung der Reaktionsprodukte mittels Gelfiltration auf einer Biogel P 2 Säule (Fa.Biorad.) <400mesh, Durchmesser 20mm, Länge 1170 mm, Flussgeschwindigkeit 12 ml pro Stunde, mit Ultraviolettabsorptionsmessung bei 280 nm, und einem Fraktionskollektor (je 20 min/Frak.) wurde durchgeführt. Alle Fraktionen der Gelfiltration wurden an einem, in einem Organbad aufgehängten Längsmuskel-Plexus-Myentericus Präparat auf ihre opiatartige Wirkung getestet. Für diesen Test wurden die einzelnen Fraktionen unter Vakuum eingedampft.

Nach der Aufnahme der Rückstände in Wasser wurden Teile dieser Proben dem Organbad zugesetzt und auf ihre hemmende Wirkung auf die elektrisch stimulierten Kontraktionen des Meerschweinchendarmpräparates getestet. Die hemmende Wirkung der zu testenden Stoffe wurde als opiatspezifisch betrachtet, wenn diese nach Zugabe des spezifischen Opiatantagonisten Naloxon aufgehoben wurden und nach weiterem Zusatz von Naloxon keine Hemmung durch die Probe mehr hervorgerufen wurde. Präparation und elektrische Stimulation des mensetzung nach Hydrolyse ergab die richtige molare tionu. elektrische Stimulation des Meerschweinchendarms (Frequenz 0,1 Hz, Pulse 60 V, 0,5 ms) wurden durchgeführt, wie von Kosterlitz et al. [Kosterlitz H.W., Lydon, R.J., Watt, A. J. Brit. J. Pharmacol. 39, 398-413 (1970)] und Schulz und Goldstein [Schulz R., Goldstein, A. J. Pharmacol. exp. Ther. 183, 404-410 (1972)] beschrieben.

Die opiataktiven Fraktionen wurden zum Trocknen gebracht.

Eine nach der sauren Hydrolyse durchgeführte Aminosäureanalyse der opiataktiven Fraktion ergab ein richtiges molares Aminosäureverhältnis entsprechend den beiden Peptiden D-Ala$^2$-β-Casomorphin-5 und D-Pro $^2$-β-Casomorphin-5.

Es sei darauf hingewiesen, dass bereits die Aktivierung des opiatinaktiven Tetrapeptids (Stufe 4 b) durch Tyrosinkopplung in ein opiataktives Peptid den Nachweis der erfolgreichen Synthese darstellt.

*Herstellung von Z-D-Ala (analog Z-D-Pro)*

10 mM D-Ala (891 mg), in 5 ml 2n NaOH, werden unter Eiskühlung und kräftigen Rühren mit 3 ml 50% Chlorameisensäurebenzylester in Toluol und mit 5,16 ml 2n NaOH behandelt.

Die Lösung wird mit 5,33 ml 2n HCl angesäuert und ausgiebig mit Ethylacetat extrahiert. Die abgetrennte Ethylacetat-Phase wird mit HCl (verd.) und Wasser gewaschen und schliesslich mit viel $KHCO_3$-Lösung extrahiert. Die vereinigten wässrigen Auszüge werden auf pH 1,5 mit HCl gebracht und mit Ethylacetat extrahiert. Die Ethylacetat-Phase wird wiederum mit verdünnter HCl gewaschen. Nach einem weiteren Waschvorgang mit Wasser wird über Natriumsulfat getrocknet. Nach dem Abdampfen des Lösungsmittels bleibt das gewünschte Endprodukt zurück.

Nachweis der Stabilität der opiatartigen Wirkung bei Inkubation mit Rattengehirnhomogenat des D--Ala$^2$-β-Casomorphin-5.

Zwei, durch Dekapitation getöteten, Ratten (männl.Sprague Dawley, 220 g Gewicht) wird rasch das Gehirn, einschliesslich Kleinhirn, entnommen und in einen, eisgekühlten, mit 7 ml 0,05 M $NaH_2PO_4$ Puffer gefüllten, Homogenisator (Nr. S. 587, Vol. 35 ccm, Braun, Melsungen) eingebracht. Die Homogenisation erfolgt dann bei einer Umdrehungszahl von 850 U/min unter zehnmaligem Auf- und Abbewegen des Homogenisatorstempels. Ein kleiner Teil des Homogenats wird entnommen und auf Eis gelagert. Der Rest wird bei 5°C 15 Minuten lang bei 28000 × g zentrifugiert. Sowohl das Homogenat vor der Zentrifugation als auch der klare Überstand nach der Zentrifugation werden für die nachfolgenden Inkubationen verwendet.

Alle Inkubationen der Substanzen mit Hirnhomogenat und -überstand erfolgen bei 37°C. Die jeweilige Inkubationszeit wird durch 10 minütiges Erhitzen bei einer Temperatur von 95°C beendet. Dieses Erhitzen auf 95°C dient dazu, noch vorhandene Enzyme zu zerstören, welche das biologische Nachweissystem für die opiatartige Wirkung beeinträchtigen könnten.

1-2 mg jeweils von β-Casomorphin-5 und dem erfindungsgemässen D-Ala$^2$-β-Casomorphin-5 werden in 50 ul $H_2O$ aufgenommen und mit je 300 ul Gehirnhomogenat und -überstand vermischt. Je eine Probe wird vor dem Inkubieren bei 37°C sofort auf 95°C erhitzt. Diese Proben waren also nur sehr kurze Zeit mit den aktiven Enzymen in Kontakt und deren Gehalt an opiatartiger Wirkung dient als Bezugswert bei Zeitpunkt Null.

Nach jeweils 15, 30, 60 und 120 Minuten werden weitere Proben der beiden inkubierten Opiate dem Inkubator entnommen und auf 95°C erhitzt.

Als Nachweissystem, welches die Opiatmengen auch quantitativ erfasst, dient der auf Seite 5 beschriebene Meerschweinchendarmtest.

Ein Test auf den Gehalt an opioiden Material in den einzelnen Gefässen ergab, dass die Gefässe welche nur kurze Zeit (Nullwert) den Enzymen ausgesetzt waren, für beide der eingesetzten Opiate den gleichen Gehalt an Aktivität aufwiesen. Aber bereits nach 30 Minuten zeigten die Gefässe welche das β-Casomorphin-5 enthielten keine opiatartige Wirkung mehr, während jedoch der volle Gehalt an opiatartiger Wirkung beim D-Ala$^2$-β-Casomorphin-5 sowohl nach 30 Minuten, als auch nach 120 Minuten Inkubation nachweisbar war. Das Peptid mit der D-Aminosäure zeigt somit eine erstaunliche Stabilität gegenüber von peptidspaltenden Enzymen.

Mit den D-Ala$^2$- und D-Pro$^2$-β-Casomorphinen konnte bei systemischer Applikation (intravenöse u. subcutane Injektion) eine analeptische Wirkung erzielt werden, was mit den entsprechenden L-Verbindungen nicht möglich war. Die Dosierung lag, entsprechend der geringeren Wirkung als Morphin, höher.

Analog dem oben aufgeführten Inkubationsbeispiel wurde beim Test der Stabilität der opiatartigen Wirkung gegenüber Blutplasma verfahren. Dabei wurden statt der 300 µl Homogenat 300 µl frisch ge-

wonnenes Rattenblutplasma eingesetzt. Wieder zeigte sich, dass das β-Casomorphin-5 ohne die D-Aminosäure bereits nach 30 Minuten zerstört ist, während das D-Ala$^2$-β-Casomorphin-s seine volle Wirkung behalten hat.

Die erfindungsgemässen Arzneimittel und Tierarzneimittel sind pharmazeutische Zubereitungen, die neben nichttoxischen, inerten, pharmazeutisch geeigneten Trägerstoffen einen oder mehrere erfindungsgemässe Wirkstoffe enthalten, oder die aus einem oder mehreren erfindungsgemässen Wirkstoffen bestehen.

Zur vorliegenden Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie Füll- und Streckmittel (z.B. Stärken, Milchzucker, Rohrzucker, Glukose, Mannit und Kiselsäure), Bindemittel (z.B. Carboxymethylcellulose, Alginate, Gelatine, Polyvinylpyrrolidon), Feuchthaltemittel (z.B. Glycerin), Sprengmittel (z.B. Agar-Agar, Calciumcarbonat und Natriumbicarbonat), Lösungsverzögerer (z.B. Paraffin) und Resorptionsbeschleuniger (z.B. quarternäre Ammoniumverbindungen), Adsorptionsmittel (z.B. Kaolin und Bentonit) und Gleitmittel (z.B. Talkum, Calcium- und Magnesiumstearat und feste Polyethylenglykole) oder Gemische der aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert, abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen zur Erzielung eines Retardeffektes.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten (z.B. tierische und pflanzliche Fette, Wachse, Paraffine, Stärken, Tragant) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, Öle, insbesondere Baumwollsaatöl, Erdnussöl, Maiskeimöl, Olivenöl und Sesamöl, Glycerin, Polyethylenglykole oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel (z.B. Wasser, Ethylalkohol, Propylenglykol), Suspendiermittel (z.B. ethoxylierte Isostearylalkohole, Polyoxyethylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, appliziert werden.

Im allgemeinen ist es in der Humanmedizin als vorteilhaft anzusehen, den oder die erfindungsgemässen Wirkstoffe in Gesamtmengen von etwa 5 bis 500, vorzugsweise 50 bis 250 mg je 24 Stunden, gegebenenfalls in Form mehrerer Einzelgaben zur Erzielung der gewünschten Ergebnisse, zu verabreichen. Eine Einzelgabe enthält den oder die erfindungsgemässen Wirkstoffe, vorzugsweise in Mengen von etwa 10 bis etwa 300, insbesondere 50 bis 200 mg/Dosis. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung u. der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muss.

Die Festlegung der jeweils erforderlichen optimalen Dosierung und Applikationsart der Wirkstoffe kann durch jeden Fachmann aufgrund seines Fachwissens leicht erfolgen.

**Ansprüche**

1. Pharmakologisch aktive Peptide der Formel:

Tyr[1]-X-A-Pro-B-Pro-C.....

wobei Tyr gleich der Aminosäure Tyrosin und Pro gleich der Aminosäure Prolin ist. A, B, C..... ist jede beliebige Aminosäure der stereochemischen L-Form. X ist jede beliebige Aminosäure der D-Form.

2. Pharmakologisch aktive Peptide nach Anspruch 1, dadurch gekennzeichnet, dass die Peptide die folgenden Formeln aufweisen:

Tyr[1]-X-A
Tyr[1]-X-A-Pro
Tyr[1]-X-A-Pro-B
Tyr[1]-X-A-Pro-B-Pro
Tyr[1]-X-A-Pro-B-Pro-C
Tyr[1]-X-A-Pro-B-Pro-C-Pro
Tyr[1]-X-A-Pro-B-Pro-C-Pro-D.....

A, B, C, D..... kann jede beliebige L-Aminosäure sein, auch Prolin und Tyrosin. X kann jede beliebige D-Aminosäure sein.

3. Pharmakologisch aktive Peptide nach einem der Ansprüche 1-2, dadurch gekennzeichnet, dass

X: D-Prolin
A: L-Phenylalanin
B: L-Glycin
C: L-Isoleucin
D: L-Asparagin.

ist.

4. Pharmakologisch aktive Peptide nach einem der Ansprüche 1-3, dadurch gekennzeichnet, dass X gleich D-Alanin, D-Threonin oder D-Valin ist.

5. Pharmakologisch aktive Peptide nach einem der Ansprüche 1-4, dadurch gekennzeichnet, dass

a) das endständige L-Tyrosin der allgemeinen Formel:

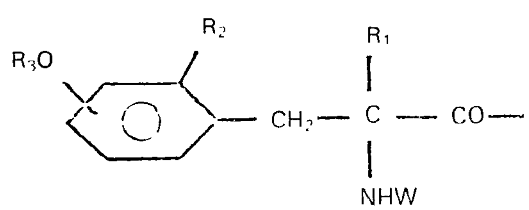

vorliegt, wobei im einzelnen bedeuten:

$R_1$ für Wasserstoff oder eine Alkylgruppe mit 1 bis 4 C-Atomen,

$R_2$ für Wasserstoff oder zusammen mit $R_1$ für eine Äthylenbrücke,

$R_3$ für Wasserstoff, eine Alkylgruppe mit 1 bis 4 C-atomen oder eine $R_4CO$-Gruppe,

$R_4$ für einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die $R_3O$-Gruppe sich in meta- oder para-Stellung zum

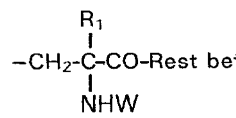 befindet,

W für Wasserstoff, Alkyl mit 1 bis 5 C-Atomen, Alkenyl mit 3 bis 5 C-Atomen, Cyclopropylmethyl, Cyclobutylmethyl, $R_4CO$-, H-Arg-, H-Lys-, H-Phe- oder H-Tyr,

b) das L-Phenylalanin der allgemeinen Formel vorliegt:

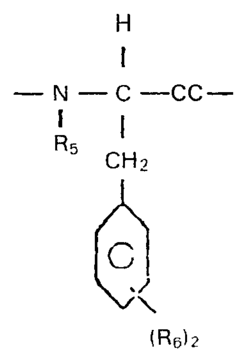

wobei im einzelnen bedeuten:

$R_5$ für Wasserstoff oder Alkyl mit 1 bis 4 C-Atomen,

$R_6$ für Wasserstoff, Fluor, Chlor, Brom, Nitro, Alkyl mit 1 bis 4 C-Atomen oder Akoxy mit 1 bis 4 C-Atomen, für 1 oder 2

c) das L-Prolin (an den Positionen der Aminosäure 4, 6, 8..... des Peptids) der allgemeinen Formel:

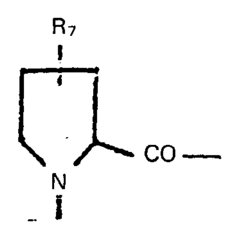

vorliegt, wobei im einzelnen

$R_7$ für Wasserstoff, eine Hydroxygruppe, eine Alkyl- oder Alkoxygruppe mit 1 bis 4 C-Atomen steht.

– am Stickstoff eine Alkyl- oder Alkoxygruppe mit 1 bis 4 C-Atomen gebunden ist.

– eine oder mehrer Ketogruppen in den Ring eingeführt sind.

d) die endständige, C-terminale Aminosäure als Amid oder Ester vorliegt.

(Die C-terminale Aminosäure liegt in der Peptidkette rechts von Tyr.)

6. Pharmakologisch aktive Peptide nach einem der Ansprüche 1-5, dadurch gekennzeichnet, dass, die C-terminale Aminosäure eines ungeradzahligen Peptids Methionin ist.

7. Pharmakologisch aktive Peptide nach einem der Ansprüche 1-6, dadurch gekennzeichnet, dass diese Penta-, Hepta- oder Nonapeptide sind.

8. Pharmakologisch aktive Peptide nach einem der Ansprüche 1-7, dadurch gekennzeichnet, dass das endständige Methionin als -Met(O)-OH der Formel:

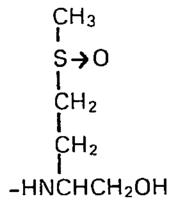

vorliegt.

9. Pharmakologisch aktive Peptide nach einem der Ansprüche 1-8, dadurch gekennzeichnet, dass diese mit einem in der Peptidchemie üblichen Verfahren synthetisiert werden.

10. Pharmakologisch aktive Peptide nach einem der Ansprüche 1-9, dadurch gekennzeichnet, dass das Knüpfen der Peptidbindung zwischen den einzelnen Aminosäuren und/oder den kleineren Peptidfragmenten mit den üblichen Methoden erfolgt.

11. Arzneimittel und Tierarzneimittel, dadurch gekennzeichnet, dass diese die in Anspruch 1-10, charakterisierten, pharmakologisch aktiven Peptide und/oder deren Derivate und/oder deren Säureadditionssalze und/oder deren Metallkomplexe enthalten.

**Revendications**

1. Des peptides pharmacologiques actives de formule

Tyr[1]-X-A-Pro-B-Pro-C.....

dans laquelle Tyr est l'amino-acide tyrosine et Pro est l'amino-acide proline (de la configuration stereochimique L). X est chaque quelconque amino-acide de la configuration stereochimique D.

2. Des peptides pharmacologiques actives suivant revendication 1, caractérisés en ce que les peptides ont les formules suivantes:

Tyr[1]-X-A
Tyr[1]-X-A-Pro
Tyr[1]-X-A-Pro-B
Tyr[1]-X-A-Pro-B-Pro
Tyr[1]-X-A-Pro-B-Pro-C
Tyr[1]-X-A-Pro-B-Pro-C-Pro
Tyr[1]-X-A-Pro-B-Pro-C-Pro-D.....

A, B, C, D peut être chaque quelconque amnino-acide de la configuration chimique L, aussi proline et tyrosine. X peut être chaque quelconque D-amino-acide.

3. Des peptides pharmacologiques actives suivant revendications 1 et 2, caractérisés en ce que

X est D-proline
A est L-phénylalanine
B est L-glycine
C est L-isoleucine
D est L-asparagine.

4. Des peptides pharmacologiques actives suivant des revendications 1 à 3, caractérisés en ce que X est egal D-alanine, D-threonine ou D-valine.

5. Des peptides pharmacologiques actives suivant des revendications 1 à 4, caractérisés en ce que

a) le L-tyrosine de la formule générale est positionné au bout et il est présent.
Dans la formule générale

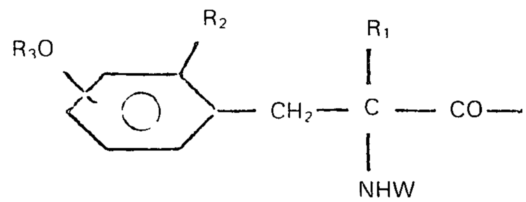

est indique en detail

$R_1$ est un atome d'hydrogène ou un groupe alkyle en $C_1$ à $C_4$,
$R_2$ est un atome d'hydrogène ou en ensemble avec $R_1$ une liaison d'éthylene,
$R_3$ est un atome d'hydrogène, un groupe alkyle en $C_1$ à $C_4$ ou un groupe de $R_4CO$-,
$R_4$ est un résidu d'alkyle, qui est saturé ou non saturé, en chaîne droite ou branchée en $C_1$ à $C_{17}$, un résidu de phényle ou de phénylalkyle en $C_7$ à $C_{12}$ dans lequel les résidus de phényle peuvent être substitués par 1 ou 2 substituents de la série d'halogènes, alkyle en $C_1$ à $C_4$ ou alkoxy en $C_1$ à $C_4$,
où le groupe de $R_3O$ est en meta ou para postion à

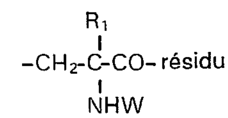

dans laquelle
W est un atome d'hydrogène, alkyle en $C_1$ à $C_5$, alcényle en $C_3$ à $C_5$, cyclopropylméthyle, cyclobutylméthyle, $R_4CO$-, H-arg-, H-lys-, H-phé-, ou H-tyr-,

b) le L-phénylalanine de formule générale

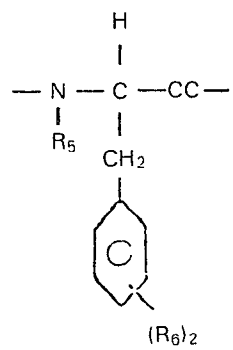

est présent, dans laquelle l'intention individuelle est:

$R_5$ est un atome d'hydrogène ou alkyle en $C_1$ à $C_4$,

$R_6$ est un atome d'hydrogène, fluorine, chlorine, bromine, nitro, alkyle en $C_1$ à $C_4$ ou alkoxy en $C_1$ à $C_4$, Z est 1 ou 2.

c) le L-proline (en des postions d'amino-acide 4, 6, 8..... du peptide) de formule générale

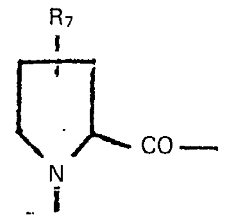

est présent, dans laquelle l'intention individuelle est

$R_7$ représente un atome d'hydrogène, un groupe d'hydroxy, un groupe d'alkyle ou un groupe d'alkoxy en $C_1$ à $C_4$

— à nitrogène est attaché un groupe d'alkyle ou un groupe d'alkoxy en $C_1$ à $C_4$

— un groupe ou plusieurs groupes de keto sont introduit dans le rond.

d) l'amino-acide C-terminal, positionné au bout, est présent qu'un amide ou ester. (L'amino-acide C-terminal est positionné à droite du tyr dans la chaîne du peptide.)

6. Des peptides pharmacologiques actives suivant l'une des revendications 1 à 5, caractérisés en ce que l'amino-acide C-terminal d'un peptide impair est méthionine.

7. Des peptides pharmacologiques actives suivant l'une des revendications 1 à 6, caractérisés en ce que ces sont des penta- hepta- ou nona-peptides.

8. Des peptides pharmacologiques actives suivant l'une des revendications 1 à 7, caractérisés en ce que le méthionine, positionné au bout, est présent qu'un -Met(O)-OH de formule

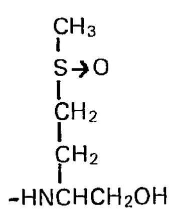

9. Des peptides pharmacologiques actives suivant l'une des revendications 1 à 8, caractérisés en ce que ces peuvent être préparés par un procédé de préparation classique de la chimie des peptides.

10. Des peptides pharmacologiques actives suivant l'une des revendications 1 à 9, caractérisés en ce que le couplage de la liaison peptidique entre les amino-acides particuliers et/ou entre plus petits fragments peptidiques résulte d'une méthode classique.

11. Des médicaments humains et des médicaments vétérinaires, caractérisés en ce que ces contient les peptides pharmacologiques actives et/ou leurs dérivatives et/ou leurs sels d'addition d'acides et/ou leurs complexes métalliques, comme caractérisés dans les revendications 1 à 10.

1. Pharmacologically active peptides of the formula:

$Tyr^1$-X-A-Pro-B-Pro-C.....

in which $Tyr^1$ is equal to the amino acid tyrosine and Pro is equal to the amino acid proline. A, B, C..... is any amino acid (of the stereochemical L-form). X is any amino acid of the D-form.

2. Pharmacologically active peptides according to claim 1, characterized in that the peptides have the following formulas:

$Tyr^1$-X-A
$Tyr^1$-X-A-Pro
$Tyr^1$-X-A-Pro-B
$Tyr^1$-X-A-Pro-B-Pro
$Tyr^1$-X-A-Pro-B-Pro-C
$Tyr^1$-X-A-Pro-B-Pro-C-Pro
$Tyr^1$-X-A-Pro-B-Pro-C-Pro-D.....

A, B, C, D..... can be any L-amino acid, such as proline and tyrosine. X can be any D-amino acid.

3. Pharmacologically active peptides according to one of the claims 1 to 2, characterized in that

X is D-proline
A is L-phenylalanine
B is L-glycine
C is L-isoleucine
D is L-asparagine.

4. Pharmacologically active peptides according to one of the claims 1 to 3, characterized in that X equals D-alanine, D-threonine or D-valine.

5. Pharmacologically active peptides according to one of the claims 1 to 4, characterized in that

a) the end-positioned L-tyrosine of the general formula is present

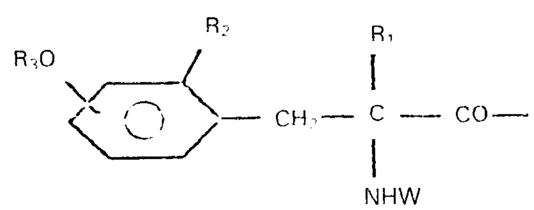

wherein the individual meaning is:

$R_1$ for hydrogen or an alkyl group with 1 to 4 C-atoms,

$R_2$ for hydrogen or together with $R_1$ for an ethylene bond,

$R_3$ for hydrogen, an alkyl group with 1 to 4 C-atoms or a $R_4CO$-group,

$R_4$ for a saturated or unsaturated straight or branched chain alkyl residue with 1 to 17 C-atoms, a phenyl residue or a phenylalkyl residue with 7 to 12 C-atoms, in which the phenyl residues can be substituted by 1 or 2 substituents of the halogen series, alkyl with 1 to 4 C-atoms or alkoxy with 1 to 4 C-atoms,

in which the $R_3O$ group is in meta or para position to

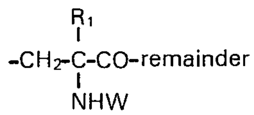

W for hydrogen, alkyl with 1 to 5 C-atoms, alkenyl with 3 to 5 C-atoms, cyclopropylmethyl, cyclobutylmethyl, $R_4CO$-, H-Arg-, H-Lys-, H-Phe-, or H-Tyr-,

b) the L-phenylalanine of the general formula

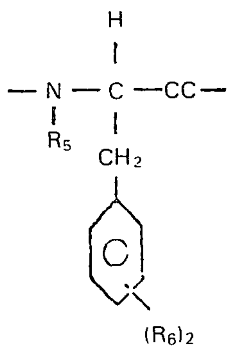

is present in which the individual meaning is:

$R_5$ for hydrogen or alkyl with 1 to 4 C-atoms,

$R_6$ for hydrogen, fluorine, chlorine, bromine, nitro, alkyl with 1 to 4 C-atoms or alkoxy with 1 to 4 C-atoms, Z for 1 or 2.

c) the L-proline (at the positions of the amino acid 4, 6, 8..... of the peptide) of the general formula

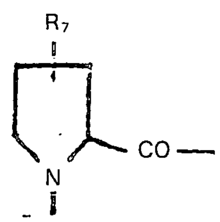

is present, in which individually

$R_7$ stands for hydrogen, a hydroxy group, an alkyl group or alkoxy group with 1 to 4 C-atoms

– at the nitrogen is bonded an alkyl group or alkoxy group with 1 to 4 C-atoms

– one or more keto groups are positioned on the ring.

d) the end-positioned, C-terminal amino acid is present as an amide or ester. (The C-terminal amino acid is positioned to the right of Tyr in the peptide chain.)

6. Pharmacologically active peptides according to one of the claims 1 to 5, characterized in that the C-terminal amino acids is of an odd numbered peptide methionine.

7. Pharmacologically active peptides according to one of the claims 1 to 6, characterized in that these are penta-peptides, hepta-peptides or nona-peptides.

8. Pharmacologically active peptides according to one of the claims 1 to 7, characterized in that the end-positioned methionine is present as -Met(O)-OH of the formula:

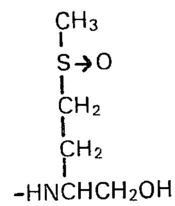

9. Pharmacologically active peptides according to one of the claims 1 to 8, characterized in that they are synthesized according to a method customary in peptide chemistry.

10. Pharmacologically active peptides according to one of claims 1 to 9, characterized in that the bonding of the peptide bond among the individual amino acids and/or the smaller peptide fragments takes place in the customary manner.

11. Medicaments and animal medicaments, characterized in that they contain the pharmacologically active peptides and/or their derivatives and/or their acid addition salts and/or their metal complexes as described in claims 1 to 10.